# EUROPEAN PATENT SPECIFICATION

(11) **EP 1 712 205 B1**
(45) Date of publication and mention of the grant of the patent: **25.01.2012**
(21) Application number: 06007891.2
(22) Date of filing: 13.04.2006
(51) Int. Cl.: A61F 2/30, A61L 27/00

(54) **Bearing implant**
Lagerimplantat
Implant porteur

(30) Priority: 15.04.2005 US 107765
(43) Date of publication of application: 18.10.2006
(73) Proprietor: Zimmer Technology, Inc., Chicago, Illinois 60606 (US)
(72) Inventor: Hodorek, Robert A., Warsaw, IN 46582 (US); Thomas, Brian H., Columbia City, IN 46725 (US)
(74) Representative: Manitz, Finsterwald & Partner GbR

(56) References cited:
- EP-A- 0 528 080
- DE-A1- 2 933 174
- US-A1- 2001 039 455
- US-A1- 2002 120 338
- US-A1- 2002 173 855
- US-A1- 2004 249 464

## Description

### FIELD OF THE INVENTION

The invention relates to a bearing implant for replacing a portion of an articular joint according to the preamble of claim 1.

### BACKGROUND

Degenerative and traumatic damage to the articular cartilage of skeletal joints can result in pain and restricted motion. Prosthetic joint replacement surgery is frequently utilized to alleviate the pain and restore joint function. During this surgery, one or more of the articulating surfaces of the joint are replaced with prosthetic bearing components. The replacement components typically include a portion for anchoring the implant adjacent to the joint and a portion for articulating with opposing joint surfaces. It is desirable for the implant to be well anchored and present a low friction, low wear articular surface.

Movable skeletal joints include abutting joint components lined with articular cartilage. Degenerative and traumatic damage to the articular cartilage can result in pain and restricted motion. Surgical joint repair is frequently utilized to alleviate the pain and restore joint function. During this surgery, a prosthetic bearing implant is interposed between the opposed bones of the joint to ease joint articulation. In some cases, the bearing implant is attached to one joint component and articulates with another joint component. In other cases, the bearing implant is in the form of a spacer that articulates with both abutting joint components. In cases of limited damage, it has been proposed to repair discrete defects on an articular surface. In cases of more extensive damage, entire joint compartments are replaced. In many cases, all of the articulating joint surfaces are replaced.

A bearing implant according to the preamble of claim 1 is known from US 2002/0173855 A1. The implant comprises a hydrogel bearing surface and anchoring pegs for anchoring the implant to a bone surface. The anchoring pegs are much stiffer and harder than the hydrogel bearing surface.

A similar bearing implant is disclosed in US 2001/0039455 A1.

DE 29 33 174 A1 discloses a bearing implant comprising a plurality of independent subunits.

US 2002/0120338 A1 relates to an implant derived from bone comprising a partially demineralized layer and a mineralized, mechanically stronger layer wherein slits are cut into the mineralized layer.

### SUMMARY

The present invention provides a bearing implant for a skeletal joint.

In one aspect, a bearing implant for replacing a portion of an articular joint surface includes a substrate including a plurality of discrete segments and a bearing surface attached to the substrate.

In another aspect, a method of repairing an articular surface of a skeletal joint includes providing a bearing implant including a substrate including a plurality of discrete segments, the segments being separated by parting lines and a bearing surface attached to the substrate, and intraoperatively shaping the implant along one or more of the parting lines to fit a surgical site.

The present invention provides a bearing implant for replacing a portion of an articular joint defined by abutting joint components.

In one aspect, the bearing implant includes a first portion and a second portion opposite the first portion joined to the first portion. At least one of the first and second portions includes a surface defined by a plurality of segments. The segments are movable relative to one another to conform to an abutting joint component. At least one of the first and second portions defines a bearing surface engageable in joint articulating relationship with an abutting joint component.

In another aspect, the bearing implant comprises a relatively flexible layer; a plurality of discrete, relatively inflexible segments separate from the flexible layer; and a way to intraoperatively attach the segments to the flexible layer to form a flexible segmented implant engageable by the abutting joint components.

In another aspect, a method comprises: inserting a first, relatively flexible first component into the joint; and joining a plurality of relatively inflexible segments to the first component in the joint to define a surface engageable with a joint component.

### BRIEF DESCRIPTION OF THE DRAWINGS

Various examples of the present invention will be discussed with reference to the appended drawings. These drawings depict only illustrative examples of the invention and are not to be considered limiting of its scope.

FIG. 1 is a top plan view of an illustrative implant which does not represent the invention; as defined in claim 1.

FIG. 2 is a side elevation view of the implant of FIG. 1 in an unflexed condition;

FIG. 3 is a bottom plan view of the implant of FIG. 1;

FIG. 4 is a side elevation view of the implant of FIG. 1 in a flexed condition;

FIG. 5 is a side elevation view of an illustrative implant similar to FIG. 1 having a segmented top bearing surface and a solid bottom surface;

FIG. 6 is a side elevation view of an illustrative implant similar to FIGS. 1 and 5 having segmented top and bottom surfaces;

FIG. 7 is a side elevation view of an illustrative implant which does not represent the invention as defined in claim 1;

FIG. 8 is a side elevation view of an illustrative implant similar to FIGS. 1, 5, and 6 having a segmented top surface and a separate bottom surface combinable intraoperatively;

FIG. 9 is a top plan view of an illustrative implant similar to FIG. 1 having an alternative parting line configuration;

FIG. 10 is a top plan view of an illustrative implant similar to FIG. 1 having an alternative parting line configuration;

FIG. 11 is a top plan view of an illustrative implant similar to FIG. 1 having an alternative parting line configuration; and

FIG. 12 is a side elevation view of an implant similar to FIG. 5 having an articular surface made up of overlapping segments..

### DESCRIPTION OF THE ILLUSTRATIVE EXAMPLES

Embodiments of a bearing implant include a bearing surface mounted to a substrate. The bearing implant may function as a replacement for damaged or diseased cartilage of a skeletal joint to sustain continued joint function. The bearing implant may be used to replace a portion of any skeletal joint including, but not limited to, joints of the hip, knee, shoulder, spine, elbow, wrist, ankle, jaw, and digits. The implant may be configured to replace a relatively small defect within the joint, an entire compartment of the joint, and/or the total joint.

The bearing surface may be made of any material suitable for articulation with natural or prosthetic opposing bearing surfaces. Preferably the bearing material is resilient to facilitate intraoperative flexing, cutting, and/or otherwise shaping of the bearing surface to fit a surgical site. The bearing surface may include polyolefins, polyesters, polyimides, polyamides, polyacrylates, polyketones, and/or other suitable materials. For example the bearing surface may include ultrahigh molecular weight polyethylene.

The bearing surface may include a hydrogel having a three dimensional network of polymer chains with water filling the void space between the macromolecules. The hydrogel may include a water soluble polymer that is crosslinked to prevent its dissolution in water. The water content of the hydrogel may range from 20-80%. The high water content of the hydrogel results in a low coefficient of friction for the bearing due to hydrodynamic lubrication. Advantageously, as loads increase on the bearing component, the friction coefficient decreases as water forced from the hydrogel forms a lubricating film. The hydrogel may include natural or synthetic polymers. Examples of natural polymers include polyhyaluronic acid, alginate, polypeptide, collagen, elastin, polylactic acid, polyglycolic acid, chitin, and/or other suitable natural polymers and combinations thereof. Examples of synthetic polymers include polyethylene oxide, polyethylene glycol, polyvinyl alcohol, polyacrylic acid, polyacrylamide, poly(N-vinyl-2-pyrrolidone), polyurethane, polyacrylonitrile, and/or other suitable synthetic polymers and combinations thereof.

The substrate provides support for the hydrogel and/or provides an anchor for the implant. The substrate may be solid or porous. The bearing surface may attach to the substrate by bonding, mechanical fasteners, porous interdigitation, and/or other suitable attachment methods. For example, the substrate may include an open porous structure in which a portion of the bearing surface is integrated to attach the bearing surface to the substrate. The substrate may be configured to be cemented in place, to be press-fit in place, to receive tissue ingrowth, and/or to be anchored to tissue in any other suitable tissue anchoring configuration. For example, the substrate may include an open porous structure for placement adjacent to body tissue to receive tissue ingrowth to anchor the implant adjacent the tissue. A porous structure may be configured to promote hard and/or soft tissue ingrowth. The porous structures may be in form of an open cell foam, a woven structure, a grid, agglomerated particles, and/or other suitable structures and combinations thereof.

The substrate may include any suitable material including, but not limited to, metals, polymers, ceramics, hydrogels and/or other suitable materials and combinations thereof. For example, a polymer substrate may include resorbable and/or non-resorbable polymers. Examples of resorbable polymers include polylactic acid polymers, polyglycolic acid polymers, and/or other suitable resorbable polymers. Examples of non-resorbable polymers include polyolefins, polyesters, polyimides, polyamides, polyacrylates, polyketones, and/or other suitable non-resorbable polymers. A metal substrate may include titanium, tantalum, stainless steel, and/or other suitable metals and alloys thereof. Preferably the substrate is relatively rigid to provide a suitable surface for hard tissue ingrowth. For example, the substrate may include a porous tantalum material having a structure similar to that of natural trabecular bone. Such a material is described in U.S. Patent No. 5,282,861 entitled "OPEN CELL TANTALUM STRUCTURES FOR CANCELLOUS BONE IMPLANTS AND CELL AND TISSUE RECEPTORS". The material is fabricated by vapor depositing tantalum into a porous matrix. The substrate may include protruding pegs or other bone engaging features to further enhance the connection of the substrate to tissue.

Tissue growth promoting substances may be included in the substrate and/or added at the time of surgery. Examples of tissue promoting substances include hydroxyapitite, particulate bone, bone growth proteins, autologous tissue derived growth factors, bone marrow aspirate, stem cells, and/or other tissue growth promoting substances.

The substrate may be formed into discrete segments to facilitate intraoperative flexing, cutting, tearing and/or otherwise shaping of the substrate to fit a surgical site. The segments may be formed from a continuous piece of substrate material by cutting, scoring, punching, molding, and/or otherwise forming the substrate. The segments may be completely separated or they may include some interconnecting substrate material as with a scored substrate being cut partway through between segments. The segments may be formed before or after the substrate and bearing surface are joined. For example a piece of substrate material may be joined to a bearing surface and subsequently the substrate may be cut to form discrete segments. In another example, the segments may be provided as discrete segments to which a bearing material is subsequently joined. The segments are spaced apart. The substrate material is more rigid than the bearing surface material.

The bearing surface may be formed by casting, injection molding, compression molding, machining, and/or other suitable forming processes and combinations thereof. For example, the bearing surface may be compression molded onto a porous substrate such that the bearing surface interdigitates with the substrate and is thereby joined to it.

Embodiments of a flexible segmented bearing implant include a body having opposed top and bottom portions. At least one of the top and bottom portions is configured to articulate with an abutting joint component. The bearing implant may function as a replacement for damaged or diseased cartilage of a skeletal joint to sustain continued joint function. The bearing implant may be used to replace a portion of any skeletal joint including, but not limited to, joints of the hip, knee, shoulder, spine, elbow, wrist, ankle, jaw, and digits of the hand and foot. The implant may be configured to replace a relatively small defect within the joint, an entire compartment of the joint, and/or the total joint. The abutting joint component with which the implant articulates may be another implant and/or the natural joint surface. The bearing implant has a top bearing surface and a bottom fixation surface.

The bearing surface may be made of any material suitable for articulation with natural or prosthetic opposing bearing surfaces. For example, the bearing surface may be made of metal, ceramic, polymer, hydrogel, and/or other materials The bearing surface may be flexible to facilitate intraoperative flexing, cutting, and/or otherwise shaping of the bearing surface to fit a surgical site. Flexibility may be imparted by the material used for the articular surface. For example, the bearing surface may include polymers, thin metals, and/or other suitable flexible materials. For example, polymers may include polyolefins, polyesters, polyimides, polyamides, polyacrylates, polyketones, and/or other suitable materials. For example the bearing surface may include ultrahigh molecular weight polyethylene.

Flexibility may also be imparted by segmenting the bearing surface. The segments may be in the form of polygons, circles, ellipses, freeform curves, and/or other suitable shapes. The segments may be in the form of elongated strips, short segments, and/or other suitable shapes. The segments may be arranged in linear patterns, curved patterns, and/or other suitable patterns. The segments may be formed from a continuous piece of bearing material by cutting, scoring, punching, molding, and/or otherwise forming the bearing surface. The segments may be completely separated or they may include some interconnecting and/or overlapping bearing material. The segments may be joined to a separate opposing portion. For example, the top surface of the implant may be defined by segments joined to the opposing bottom portion of the implant to support the segments. The segments may be formed before or after the opposing portions are joined. For example a piece of bearing material may be joined to the opposing portion and subsequently the bearing surface may be formed into discrete segments. In another example, the segments may be provided as discrete segments to which the opposing portion is subsequently joined. The segments may overlap one another but are spaced apart. The bearing material may be relatively more rigid than the opposing surface material. For example, the segments may have rigid, hard bearing surfaces and the opposing portion may be relatively flexible such that the implant conforms to the underlying anatomic surface. The opposing portion may permit relative motion of the bearing surface segments during articulation. The bearing surface segments may be able to pivot to orient the segments relative to an abutting articular component. For example the segments may be able to rock relative to one another to orient each segment bearing surface normal to the abutting articulating component. The segments may be able to move sufficiently relative to the opposing portion to conform the segmented bearing surface to the shape of the abutting bearing component. The segments may be shaped and arranged such that the implant flexes into a predetermined shape corresponding to a desired anatomic shape. For example, the segments may be configured so that the implant flexes into a dished, channeled, ridged, and/or other suitable shape.

The bearing surface may include a lubricant to ease articulation. For example, the bearing surface may include hyaluronic acid and/or a hydrodynamically lubricated hydrogel layer. For example, the bearing surface may include hyaluronic acid impregnated into the surface. The bearing surface may include a hydrogel having a three dimensional network of polymer chains with water filling the void space between the macromolecules. The hydrogel may include a water soluble polymer that is crosslinked to prevent its dissolution in water. The water content of the hydrogel may range from 20-80%. The high water content of the hydrogel results in a low coefficient of friction for the bearing due to hydrodynamic lubrication. Advantageously, as loads increase on the bearing component, the friction coefficient decreases as water forced from the hydrogel forms a lubricating film. The hydrogel may include natural or synthetic polymers. Examples of natural polymers include polyhyaluronic acid, alginate, polypeptide, collagen, elastin, polylactic acid, polyglycolic acid, chitin, and/or other suitable natural polymers and combinations thereof. Examples of synthetic polymers include polyethylene oxide, polyethylene glycol, polyvinyl alcohol, polyacrylic acid, polyacrylamide, poly(N-vinyl-2-pyrrolidone), polyurethane, polyacrylonitrile, and/or other suitable synthetic polymers and combinations thereof.

The bearing surface may attach to the opposite portion by bonding, mechanical fasteners, porous interdigitation, and/or other suitable attachment methods. For example, the opposite portion may include an open porous structure in which a portion of the bearing surface is integrated to attach the bearing surface to the opposite portion.

A fixation surface may fix the implant to an underlying anatomic surface to support the bearing surface in generally fixed relationship relative to the surgical site.
The fixation surface may be solid or porous. The fixation surface may be configured to be cemented in place, to be press-fit in place, to receive tissue ingrowth, and/or to be anchored to tissue in any other suitable tissue anchoring configuration. For example, the fixation surface may include an open porous structure for placement adjacent to body tissue to receive tissue ingrowth to anchor the implant adjacent the tissue. A porous structure may be configured to promote hard and/or soft tissue ingrowth. The porous structures may be in form of an open cell foam, a woven structure, a grid, agglomerated particles, and/or other suitable structures and combinations thereof.

The fixation surface may include any suitable material including, but not limited to, metals, polymers, ceramics, hydrogels and/or other suitable materials and combinations thereof. For example, a polymer fixation surface may include resorbable and/or non-resorbable polymers. Examples of resorbable polymers include polylactic acid polymers, polyglycolic acid polymers, and/or other suitable resorbable polymers. Examples of non-resorbable polymers include polyolefins, polyesters, polyimides, polyamides, polyacrylates, polyketones, and/or other suitable non-resorbable polymers. A metal fixation surface may include titanium, tantalum, stainless steel, and/or other suitable metals and alloys thereof. The fixation surface may provide a suitable surface for hard tissue ingrowth. For example, the fixation surface may include a porous tantalum material having a structure similar to that of natural trabecular bone. Such a material is described in U.S. Patent No. 5,282,861 entitled "Open Cell Tantalum Structures For Cancellous Bone Implants And Cell And Tissue Receptors". The material is fabricated by vapor depositing tantalum into a porous matrix. The fixation surface may include protruding pegs or other bone engaging features to further enhance the connection of the fixation surface to tissue.

Tissue growth promoting substances may be included in the implant and/or added at the time of surgery. Examples of tissue promoting substances include hydroxyapitite, particulate bone, bone growth proteins, autologous tissue derived growth factors, bone marrow aspirate, stem cells, and/or other tissue growth promoting substances.

The fixation surface may be flexible to facilitate intraoperative flexing, cutting, and/or otherwise shaping of the fixation surface to fit a surgical site. Flexibility may be imparted by the material used for the fixation surface. For example, the fixation surface may include polymers, thin metals, hydrogels, and/or other suitable flexible materials.

Flexibility may also be imparted by segmenting the fixation surface. The segments may be in the form of polygons, circles, ellipses, freeform curves, and/or other suitable shapes. The segments may be in the form of elongated strips, short segments, and/or other suitable shapes. The segments may be arranged in linear patterns, curved patterns, and/or other suitable patterns. The segments may be formed from a continuous piece of fixation surface material by cutting, scoring, punching, molding, and/or otherwise forming the fixation surface. The segments may be completely separated or they may include some interconnecting and/or overlapping fixation material. The segments may be formed before or after the bearing surface and fixation surface are joined. For example a piece of bearing material may be joined to a piece of fixation surface material and subsequently the fixation surface may be cut to form discrete segments. In another example, the segments may be provided as discrete segments to which a bearing material is subsequently joined. The segments may overlap one another, abut one another but they are spaced apart. The fixation surface material may be relatively more rigid than the bearing material. For example, the segments may be relatively rigid and the bearing surface may be relatively flexible such that the fixation surface segments flex relative to one another due to bending of the bearing surface. The segments may be shaped and arranged such that the implant flexes into a predetermined shape corresponding to a desired anatomic shape. For example, the segments may be configured so that the implant flexes into a dished, channeled, ridged, and/or other suitable shape.

The top and bottom portions may be joined with an intermediate layer of flexible material. An intermediate layer may be molded between the top and bottom portions. The top and bottom surfaces may both be segmented. For example, the top portion may define a bearing surface including segments having a hard, smooth bearing surface and the bottom portion may define a fixation surface including segments having a porous bone ingrowth configuration. In another example, the top and bottom portions may both define bearing surfaces. The flexible layer may be sufficiently thick and resilient that the top and bottom surface segments may flex relative to one another. For example, the bottom segmented surface may flex to conform to the shape of an underlying joint component, such as a bone surface, and the top segmented surface may flex independently of the bottom surface to conform to an abutting articulating joint component.

The implant may be formed of discrete segments in which each segment includes a top and bottom joint contacting surface and the discrete segments may be joined together with a flexible material to allow flexing of the implant. For example each segment may have a smooth, relatively non-porous top bearing surface and a rough, relatively porous bone ingrowth bottom surface and the segments may be joined with a flexible material between their sides to permit relative motion of the segments.

The top and bottom surfaces may be joined during manufacture and provided as a flexible implant shaped for a specific anatomic application. The implant may be able to be shaped intraoperatively to fit a surgical site such as by flexing, cutting, and/or tearing the implant. The implant may also be provided as separate top and bottom portions that are joined intraoperatively. For example, one of the components may be supplied as discrete segments and the other supplied as a continuous layer for joining intraoperatively. For example, the bottom surface may be provided as discrete fixation segments that are positionable in a desired pattern on an underlying anatomic surface and the top surface may be provided as a continuous flexible bearing layer that is joined to the segments intraoperatively to form the implant. In another example, the bottom surface may be provided as a continuous flexible layer that is positionable on the underlying anatomic surface and the top surface may be provided as discrete bearing surface segments that are placed on the bottom surface intraoperatively in a desired pattern. In another example, both the top and bottom surface may be provided as discrete segments joined together intraoperatively by a flexible intermediate layer.

The top and bottom surfaces may be joined intraoperatively with mechanical fasteners, adhesives, and/or other suitable joining methods. Mechanical fasteners may include posts, screws, teeth, hook and loop arrangements, and/or other suitable mechanical fasteners. Adhesives may include biologic adhesives, synthetic adhesives, one-part adhesives, multi-part adhesives, heat activated adhesives, light activated adhesives, and/or other suitable adhesives. For example, adhesives may include fibrin adhesive, cyanoacrylate adhesive, bone cement, epoxy, and/or other suitable adhesive.
For example, the top and bottom surfaces may be joined by coating one with a first part of a two-part adhesive and the other with a second part, or an activator, of the two-part adhesive and then contacting them intraoperatively to cause the adhesive to cure and join them. In another example, one of the top and bottom surfaces may include a hook arrangement and the other may include a loop arrangement that fasten together on contact. Where the implant includes segments in which each segment includes both a top and a bottom operative surface, the segments may be intraoperatively joined by employing the joining method between the sides of adjacent segments.

A fixation surface may be joined to the underlying anatomic surface with mechanical fasteners, adhesives, bone ingrowth, and/or other suitable joining method. Mechanical fasteners may include posts, screws, teeth, hook and loop arrangements, and/or other suitable mechanical fasteners. Adhesives may include fibrin adhesive, cyanoacrylate, bone cement, epoxy, and/or other suitable adhesive.

Bearing and fixation surfaces may be formed by casting, molding, extruding, machining, and/or other suitable forming processes and combinations thereof.

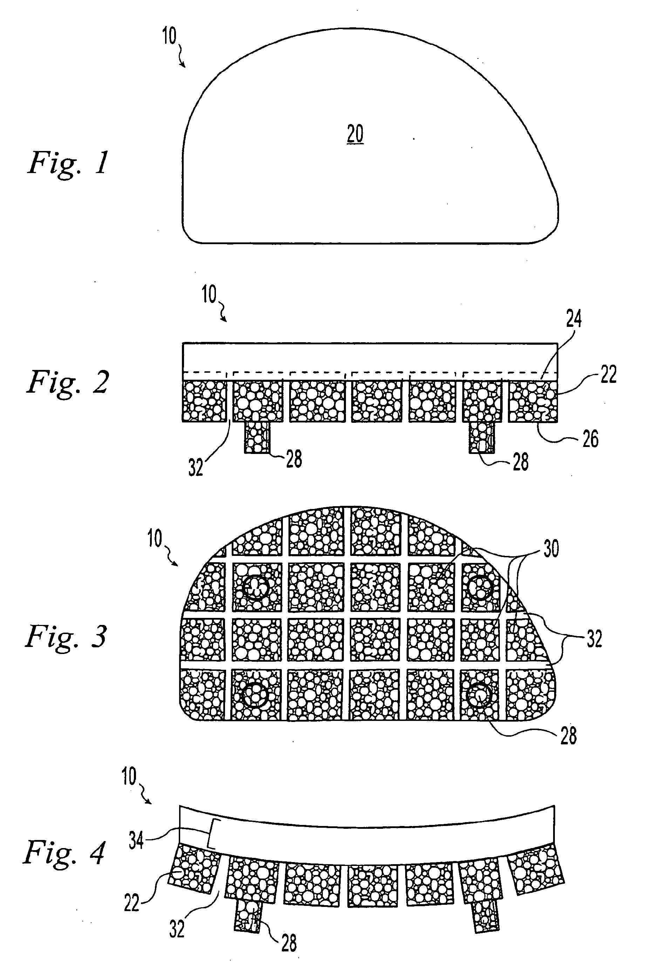

FIGS. 1-4 depict an illustrative example of a bearing implant not representing the invention as defined in claim 1. The illustrative implant 10 is in the form of a unicondylar tibial knee joint prosthesis. However, it is within the scope of the invention for the bearing implant 10 to be configured to replace a small portion of the tibial articular bearing surface, to replace an entire compartment of the tibial articular bearing surface (as shown), to replace both compartments of the tibial articular bearing surface, to replace a portion of the femoral condyles of the knee joint, and/or to replace any amount of any bearing surface in any skeletal joint. The implant 10 includes a top, or proximal, portion defining a bearing surface 20 to receive an abutting portion of the joint in articulating relationship and a distal, or bottom, portion 22. The bottom portion 22 preferably includes a first porous region 24 into which a portion of the top surface 20 is interdigitated to connect the top surface 20 to the bottom surface 22. In the illustrative example, a hydrogel bearing surface 20 is molded into the pores of the first porous region 24. Preferably the bottom portion 22 includes a second porous region 26 for placement against tissue for receiving tissue ingrowth. In the illustrative example, the bottom portion 22 is porous tantalum and is porous throughout to provide first and second porous regions 24 and 26. The illustrative bottom portion 22 includes protruding pegs 28 for insertion into holes formed in an underlying bone to further enhance the connection of the bottom portion 22 to the bone.

In the illustrative example, the bottom portion 22 is formed into a grid of discrete, generally planar segments 30 separated by parting lines 32. The parting lines 32 facilitate intraoperative flexing, tearing, cutting, and/or otherwise shaping the implant 10. For example, the parting lines 32 result in a thinner region 34 along which the implant 10 is more flexible. The parting lines 32 may be relatively narrow (not shown) so that the segments 30 abut one another in an unflexed state and appear as one continuous bottom surface. In this configuration, the implant 10 will be more flexible in a direction that tends to open the parting lines 32 and be more rigid in a direction that tends to press the segments 30 together. Alternately, the parting lines 32 may be relatively wide (as shown) to provide a gap between segments 30 to facilitate flexing of the implant 10 both in directions that tend to open the parting lines 32 (FIG. 4) and in directions that tend to close the parting lines 32. The parting lines may extend all the way through the bottom portion 22 (as shown) or they may be scored only partway through the bottom portion 22. The number and shape of the segments 30 and parting lines 32 may be tailored for particular applications to enhance and/or restrict flexibility in portions of the implant 10. For example, the implant may have two segments 30 separated by a single parting line 32 allowing the two segments to flex relative to one another along the single parting line. The implant 10 may have any number of segments 30 suitable to a particular application. The segments may likewise vary in shape from a few relatively large segments to many relatively small segments. In the illustrative example, the bearing surface 20 provides a relatively flexible, lubricious bearing surface 20, while the segments 30 provide individual, relatively rigid bone mounting surfaces.

The parting lines 32 also facilitate cutting, tearing and/or otherwise shaping the bottom portion 22. The parting lines 32 present thinner regions 34 of the implant that may be more easily cut with a knife, scissors, shears, or other cutting instrument. The parting lines 32 may extend all the way through a difficult to cut bottom portion 22, such as a metal bottom portion 22 (as shown), so that only the top portion 20 need be cut intraoperatively. With some materials, the parting lines 32 may make it possible to tear away unneeded segments. The number and shape of the segments 30 and parting lines 32 may be tailored to define predetermined implant shapes corresponding to different surgical sites, differing patient anatomy, and/or different defect shapes and/or sizes. The user can selectively shape the implant along a desired parting line to match the implant shape to the particular use.

In use, the implant 10 is compared to a cartilage region that is to be repaired. The shape of the desired replacement is noted and then the implant is flexed, torn, cut and/or otherwise reshaped along the parting lines 32 to approximate the desired replacement. The implant 10 is then anchored to the underlying tissue by cementing, press fitting, and/or juxtaposing it for hard and/or soft tissue ingrowth. In the illustrative example, holes are drilled into underlying bony tissues and the pegs 28 are pressed into the holes with the segments 30 abutting the underlying bony tissues to facilitate bony ingrowth into the pegs 28 and segments 30 to anchor the implant 10.

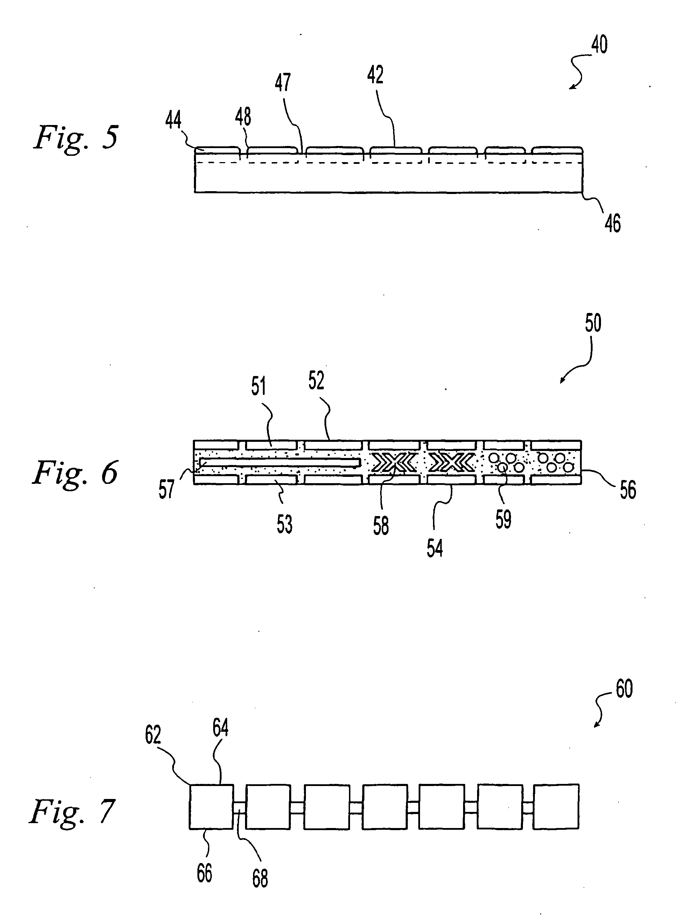

FIG. 5 illustrates an implant 40 having a segmented bearing surface 42 made of discrete bearing segments 44 embedded in a flexible bottom portion 46. There is a space 47 between adjacent segments 44 that allows independent movement of the segments 44. In the illustrative example, the bearing segments 44 are made of metal to provide a hard, wear resistant bearing surface 42. The bottom portion 46 is made of a flexible and resilient hydrogel. As an abutting bearing, such as a prosthesis or natural bone portion, articulates against the bearing surface 42, the segments 44 are free to rock and move within the hydrogel such that the bearing surface 42 conforms to the shape of the opposing bearing to provide a large contact area. The resiliency of the bottom portion 46 allows the segments 44 to conform to a variety of abutting bearing components. For example, in a knee joint, the portion of the femoral articular surface in contact with the tibia changes from a relatively larger radius in extension to a relatively smaller radius in flexion. A tibial implant made according to the implant of FIG. 5 includes segments 44 that adjust their orientation to conform to the changing radius of the femoral articular surface as the knee joint articulates. The hard bearing surface 42 of the implant resists abrasive wear. The segments 44 may vary in size and spacing to provide for more or less conformity to the opposing surface. The bottom portion 46 may be recessed below the bearing surface 42 (as shown) to protect it from wear or it may extend around the segments 44 so that it is flush with the bearing surface. The edges 48 of the segments 44 are relieved by forming a radius on the edge 48 to ensure smooth articulation.

FIG. 6 illustrates an implant 50 having a bearing surface 52 and a bottom surface 54 both made of discrete segments 51, 53 and joined by an intermediate flexible layer 56. In the illustrative example, the bearing surface 52 is made of ceramic segments 51, the bottom surface 54 is made of porous tantalum segments 53, and the intermediate layer 56 is a flexible polymer molded to and joining all of the segments into a flexible implant. For example, the intermediate layer 56 may include a hydrogel. In the illustrative implant 50 of FIG. 6, the intermediate layer 56 extends in between the bearing surface segments 51 and is flush with the bearing surface 52. Under load, the hydrogel will release fluid to lubricate the bearing surface 52.

The intermediate layer 56 may define a gradient from a harder and/or stiffer material at the surface 52 to a softer and/or less stiff material toward the bottom. The gradient may be defined by placing a softer material in the center of the intermediate material 56. Examples of suitable materials include silicones, urethanes, low density polyethylene, elastomers, and/or other suitable materials.

The intermediate layer 56 may also include a fluid. As the implant is loaded, pressure is redistributed in fluid from loaded to unloaded areas of the implant and increases conformity and contact area of the flexible implant with the abutting joint surface.

The intermediate layer 56 may define a gradient geometrically. For example, voids 57, 58, 59 may be formed in the intermediate layer to change the stiffness of the intermediate layer 56. The voids may extend under multiple segments as shown at 57 or they may be tailored for discrete segments as at 58 and 59. Any combination of voids may be utilized to achieve the desired stiffness. The voids 57, 58, 59 may be empty or filled. For example, they may be filled with a gas, liquid, a gel, and/or some other substance.

FIG. 7 illustrates an implant 60 made of discrete segments 62 having top and bottom surfaces 64, 66. In the illustrative example, the segments 62 comprise a lubricious polymer such as polyethylene and/or a hydrogel. The top and bottom surfaces 64, 66 are smooth articulating surfaces permitting articulation with opposing joint components on both the top and bottom surfaces 64, 66. Alternatively, the top surface 64 may comprise a smooth bearing surface and the bottom surface 66 may comprise fixation surface. The segments 62 are joined at their sides by flexible joiners 68 to permit the implant 60 to flex to conform to the shape of the opposing joint components. The implant of FIG. 7 forms a flexible spacer for insertion within a joint. The joiners 68 may be strands, bands, blocks, sheets, and/or other suitable shapes. The joiners 68 may be formed of metals, polymers, and/or other suitable materials. The joiners 68 may pass through the segments 62 to weave the segments 62 together. Additional porous pads (not shown) could be woven to the bottom of the implant to form a bone fixation interface.

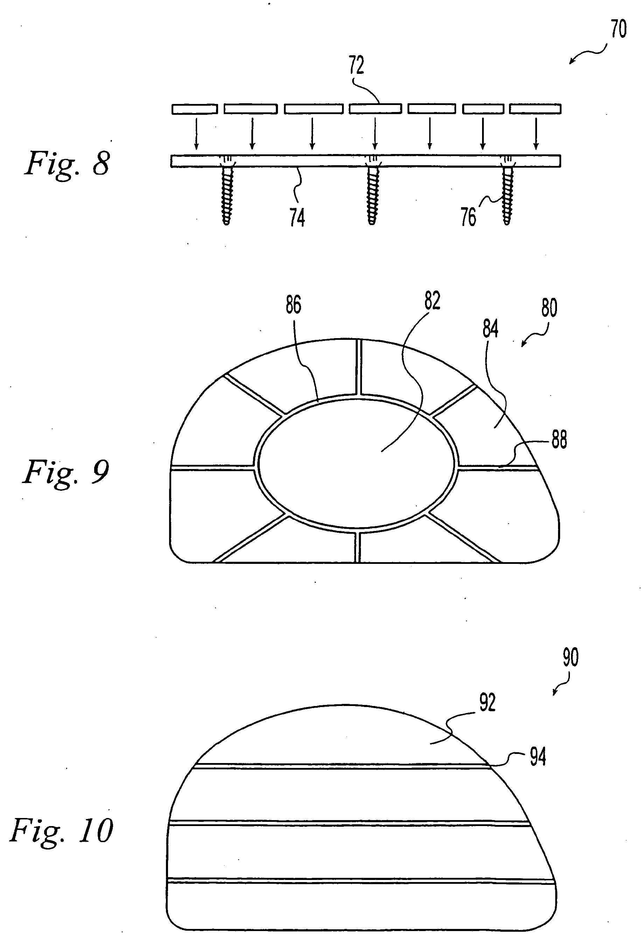

FIG. 8 illustrates an implant 70 made of discrete segments 72 defining a bearing surface and a flexible portion 74 provided separately. The flexible portion 74 is first placed in the joint. It flexibly conforms to the underlying anatomic surface and is fixed in place with illustrative bone screws 76. The segments 72 are subsequently attached to the flexible portion 74 to form an implant 70 having the desired shape. In the illustrative example, the segments 72 include a first part of a two-part adhesive system and the portion 74 includes the second part such that the segments are bonded to the flexible portion 74 after they are placed in contact with one another.

FIG. 9 illustrates an implant 80 having segments 82, 84 having varying shapes and separated with parting lines 86, 88 of varying shapes such that the implant 80 flexes into a predetermine shape. In the illustrative example, a central oval segment 82 is separate from the surrounding segments 84 by an oval parting line 86. The surrounding segments 84 are generally polygonal and separated from one another by radial parting lines 88. The implant 80 flexes into an oval dish shape. The segments may be formed on a bearing side and/or a bone fixation side of the implant.

FIG. 10 illustrates an implant 90 having segments 92 arranged as elongated, parallel strips separated by elongated parting lines 94 such that the implant 90 flexes into a predetermine cylindrical shape. The segments may be formed on a bearing side and/or a bone fixation side of the implant.

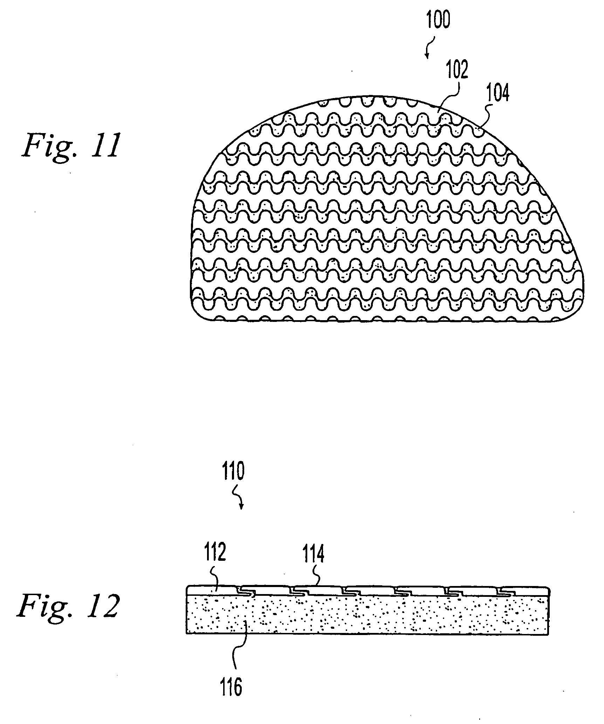

FIG. 11 illustrates an implant 100 having elongated serpentine segments 102 arranged parallel to one another. The segments 102 may be formed by corrugating, crimping, bending, molding, and/or other wise forming them into serpentine shapes. The serpentine shape of the segments 102 allows the segments to bend in multiple directions and to elongate so that the implant may flex into a variety of shapes conforming to an abutting joint surface. The segments 102 are supported in a hydrogel matrix 104 to facilitate flexing of the segments as well as to provide lubrication to the segment 102 surfaces. In the illustrative example, the segments 102 are formed of thin sections of a relatively hard material that resist abrasive wear while permitting them to flex. The matrix 104 optionally separates adjacent segments 102 to prevent them from rubbing on one another.

FIG. 12 illustrates an implant 110 having overlapping plate-like segments 112 defining a relatively smooth articular surface 114. The segments are supported by a flexible hydrogel matrix 116. The matrix may optionally extend between the overlapping portions of the segments 112 to separate the overlapping portions and prevent them from rubbing on one another.

The implant of the invention can be used in method of repairing an articular surface of a skeletal joint, the method comprising: providing a bearing implant having a substrate comprising a plurality of discrete segments, the segments being separated by parting lines; and a bearing surface attached to the substrate; intraoperatively shaping the implant along one or more of the parting lines to fit a surgical site. Preferably, shaping the implant comprises flexing the implant. Preferably, shaping the implant comprises cutting the implant along one or more of the parting lines. Preferably, shaping the implant comprises tearing the implant along one or more of the parting lines.
The bearing implant can be used in a method for placing the bearing implant in a joint, the method comprising: inserting a first, relatively flexible first component (74) into the joint; and joining a plurality of relatively inflexible segments (72) to the first component in the joint to define a surface engageable with a joint component. Preferably, the first component comprises a bone fixation component and the segments are joined to the bone fixation component to form a segmented articular surface. Preferably, the first component comprises a bearing surface component and the segments are joined to the bearing surface component to form a segmented bone fixation surface.

Although examples of a bearing implant and its use have been described and illustrated in detail, it is to be understood that the same is intended by way of illustration and example only and is not to be taken by way of limitation. The invention has been illustrated in the context of a tibial articular implant. However, the bearing implant may be configured in other shapes and for use at other locations within a patient's body. Accordingly, variations in and modifications to the bearing implant and its use will be apparent to those of ordinary skill in the art, and the following claims are intended to cover all such modifications.

## Claims

1. Bearing implant for replacing a portion of an articular joint defined by abutting joint components, the implant (40, 50, 70, 80, 90, 100, 110) comprising a first portion (46, 54, 74, 116) formed from a first material; and a second portion (42, 44, 52, 72, 82, 84, 92, 102, 114) formed from a second material, the second portion positioned opposite the first portion (46, 54, 74, 116) and joined to the first portion (46, 54, 74, 116), the second portion (42, 44, 52, 72, 82, 84, 92, 102, 114) including a plurality of discrete segments (44, 51, 53, 72, 82, 84, 92, 102, 112), each of the plurality of discrete segments (44, 51, 53, 72, 82, 84, 92, 102, 112) spaced apart from one another so that a plurality of gaps (47, 86, 88, 94) are formed between respective pairs of the discrete segments (44, 51, 53, 72, 82, 84, 92, 102, 112),
**characterized in that**
the second material is more rigid than the first material, the first portion (46, 54, 74, 116) extends into the plurality of gaps (47, 86, 88, 94) formed between the plurality of discrete segments (44, 51, 53, 72, 82, 84, 92, 102, 112), the discrete segments (44, 51, 53, 72, 82, 84, 92, 102, 112) are movable relative to one another to conform to a first abutting joint component, the plurality of discrete segments (44, 51, 53, 72, 82, 84, 92, 102, 112) of the second portion (42, 44, 52, 72, 82, 84, 92, 102, 114) each have a respective bearing surface (42, 52, 114) disposed opposite the first portion (46, 54, 74, 116), each bearing surface (42, 52, 114) directly engageable in joint articulating relationship with the first abutting joint component, and the first portion (46, 54, 74, 116) defines a fixation surface (54, 74) for mounting to a second abutting joint component defined by a bone surface.

2. Bearing implant of claim 1 **characterized in that** the first portion (46, 54, 74, 116) and the second portion (42, 44, 52, 72, 82, 84, 92, 102, 114) are joinable intraoperatively.

3. Bearing implant according to any of the preceding claims **characterized in that** the first portion (46, 54, 74, 116) comprises a continuous flexible portion fixable to bone and the segments (44, 51, 53, 72, 82, 84, 92, 102, 112) comprise discrete bearing surface segments fixable to the first portion (46, 54, 74, 116) and comprising a bearing surface (42, 52, 114) engageable with a joint component in joint articulating relationship.

4. Bearing implant according to any of the preceding claims **characterized in that** each of the plurality of discrete segments (44, 51, 53, 72, 82, 84, 92, 102, 112) is pivotable relative to the first material, each segment (44, 51, 53, 72, 82, 84, 92, 102, 112) being orientable normal to a surface of the first abutting joint component.

5. Bearing implant according to any of the preceding claims **characterized in that** the plurality of discrete segments comprises elongated strips (92, 102).

6. Bearing implant according to any of the preceding claims **characterized in that** the plurality of segments comprises a central segment (82) and a plurality of radial segments (84) extending outwardly from the central segment (82).

7. Bearing implant according to any of the preceding claims **characterized in that** the plurality of discrete segments comprises a plurality of elongated segments (92, 102) arranged parallel to one another with elongated gaps (94) between the segments.

8. Bearing implant according to any of the preceding claims **characterized in that** the plurality of discrete segments comprises a plurality of serpentine segments (102).

9. Bearing implant according to any of the preceding claims **characterized in that** the plurality of segments comprises a plurality of overlapping plate-like segments (112).

## Patentansprüche

1. Lagerimplantat zum Austausch eines Anteils eines künstlichen Gelenks, das durch angrenzende Gelenkkomponenten definiert ist, wobei das Implantat (40, 50, 70, 80, 90, 100, 110) einen ersten Abschnitt (46, 54, 74, 116), der aus einem ersten Material geformt ist; und einen zweiten Abschnitt (42, 44, 52, 72, 82, 84, 92, 102, 114) umfasst, der aus einem zweiten Material geformt ist, wobei der zweite Abschnitt gegenüberliegend dem ersten Abschnitt (46, 54, 74, 116) positioniert ist und mit dem ersten Abschnitt (46, 54, 74, 116) verbunden ist, wobei der zweite Abschnitt (42, 44, 52, 72, 82, 84, 92, 102, 114) eine Mehrzahl diskreter Segmente (44, 51, 53, 72, 82, 84, 92, 102, 112) aufweist, wobei jedes der Mehrzahl diskreter Segmente (44, 51, 53, 72, 82, 84, 92, 102, 112) voneinander so beabstandet ist, dass eine Mehrzahl von Spalten (47, 86, 88, 94) zwischen jeweiligen Paaren der diskreten Segmente (44, 51, 53, 72, 82, 84, 92, 102, 112) geformt ist,
**dadurch gekennzeichnet, dass**
das zweite Material starrer als das erste Material ist, sich der erste Abschnitt (46, 54, 74, 116) in die Mehrzahl von Spalten (47, 86, 88, 94) erstreckt, die zwischen der Mehrzahl diskreter Segmente (44, 51, 53, 72, 82, 84, 92, 102, 112) geformt sind, die diskreten Segmente (44, 51, 53, 72, 82, 84, 92, 102, 112) relativ zueinander bewegbar sind, um sich an eine erste angrenzende Gelenkkomponente anzupassen, die Mehrzahl diskreter Segmente (44, 51, 53, 72, 82, 84, 92, 102, 112) des zweiten Abschnittes (42, 44, 52, 72, 82, 84, 92, 102, 114) jeweils eine jeweilige Lagerfläche (42, 52, 114) besitzen, die gegenüberliegend dem ersten Abschnitt (46, 54, 74, 116) angeordnet ist, wobei jede Lagerfläche (42, 52, 114) direkt in Gelenkbewegungsbeziehung mit der ersten angrenzenden Gelenkkomponente in Eingriff bringbar ist und der erste Abschnitt (46, 54, 74, 116) eine Fixationsfläche (54, 74) zur Montage an einer zweiten angrenzenden Gelenkkomponente, die durch eine Knochenfläche definiert ist, definiert.

2. Lagerimplantat nach Anspruch 1,
**dadurch gekennzeichnet, dass**
der erste Abschnitt (46, 54, 74, 116) und der zweite Abschnitt (42, 44, 52, 72, 82, 84, 92, 102, 114) intraoperativ verbindbar sind.

3. Lagerimplantat nach einem der vorhergehenden Ansprüche,
**dadurch gekennzeichnet, dass**
der erste Abschnitt (46, 54, 74, 116) einen kontinuierlichen flexiblen Abschnitt umfasst, der am Knochen fixierbar ist, und die Segmente (44, 51, 53, 72, 82, 84, 92, 102, 112) diskrete Lagerflächensegmente umfassen, die an dem ersten Abschnitt (46, 54, 74, 116) fixierbar sind und eine Lagerfläche (42, 52, 114) umfassen, die mit einer Gelenkkomponente in Gelenkbewegungsbeziehung in Eingriff bringbar ist.

4. Lagerimplantat nach einem der vorhergehenden Ansprüche,
**dadurch gekennzeichnet, dass**
jedes der Mehrzahl diskreter Segmente (44, 51, 53, 72, 82, 84, 92, 102, 112) relativ zu dem ersten Material schwenkbar ist, wobei jedes Segment (44, 51, 53, 72, 82, 84, 92, 102, 112) normal zu einer Fläche der ersten angrenzenden Gelenkkomponente orientierbar ist.

5. Lagerimplantat nach einem der vorhergehenden Ansprüche,
**dadurch gekennzeichnet, dass**
die Mehrzahl diskreter Segmente längliche Streifen (92, 102) umfasst.

6. Lagerimplantat nach einem der vorhergehenden Ansprüche,
**dadurch gekennzeichnet, dass**
die Mehrzahl von Segmenten ein Zentralsegment (82) und eine Mehrzahl von Radialsegmenten (84) umfasst, die sich von dem Zentralsegment (82) auswärts erstrecken.

7. Lagerimplantat nach einem der vorhergehenden Ansprüche,
**dadurch gekennzeichnet, dass**
die Mehrzahl diskreter Segmente eine Mehrzahl länglicher Segmente (92, 102) umfasst, die parallel zueinander mit länglichen Spalten (94) zwischen den Segmenten angeordnet sind.

8. Lagerimplantat nach einem der vorhergehenden Ansprüche,
**dadurch gekennzeichnet, dass**
die Mehrzahl diskreter Segmente eine Mehrzahl von Serpentinensegmenten (102) umfasst.

9. Lagerimplantat nach einem der vorhergehenden Ansprüche,
**dadurch gekennzeichnet, dass**
die Mehrzahl von Segmenten eine Mehrzahl von Segmenten (112) im Stil sich überlappender Platten umfasst.

## Revendications

1. Implant porteur pour remplacer une portion d'un joint articulaire défini par des composants de joints en aboutement, l'implant (40, 50, 70, 80, 90, 100, 110) comprenant une première portion (46, 54, 74, 116) formée d'un premier matériau ; et une seconde portion (42, 44, 52, 72, 82, 84, 92, 102, 114) formée d'un second matériau, la seconde portion étant positionnée à l'opposé de la première portion (46, 54, 74, 116) et réunie à la première portion (46, 54, 74, 116), la seconde portion (42, 44, 52, 72, 82, 84, 92, 102, 114) incluant une pluralité de segments discrets (44, 51, 53, 72, 82, 84, 92, 102, 112), chacun de la pluralité de segments discrets (44, 51, 53, 72, 82, 84, 92, 102, 112) étant espacés les uns des autres de telle façon qu'une pluralité d'intervalles (47, 86, 88, 94) sont formés entre des paires respectives de segments discrets (44, 51, 53, 72, 82, 84, 92, 102, 112),
**caractérisé en ce que**
le second matériau est plus rigide que le premier matériau, la première portion (46, 54, 74, 116) s'étend jusque dans la pluralité d'intervalles (47, 86, 88, 94) formés entre la pluralité de segments discrets (44, 51, 53, 72, 82, 84, 92, 102, 112), les segments discrets (44, 51, 53, 72, 82, 84, 92, 102, 112) sont mobiles les uns par rapport aux autres pour se conformer à un premier composant de joint en aboutement, la pluralité de segments discrets (44, 51, 53, 72, 82, 84, 92, 102, 112) de la seconde portion (42, 44, 52, 72, 82, 84, 92, 102, 114) ont chacun une surface portante respective (42, 52, 114) disposée à l'opposé de la première portion (46, 54, 74, 116), chaque surface portante (42, 52, 114) peut être directement engagée dans une relation de joint articulé avec le premier composant de joint en aboutement, et la première portion (46, 54, 74, 116) définit une surface de fixation (54, 74) pour le montage sur un second composant de joint en aboutement défini par une surface osseuse.

2. Implant porteur selon la revendication 1, **caractérisé en ce que** la première portion (46, 54, 74, 116) et la seconde portion (42, 44, 52, 72, 82, 84, 92, 102, 114) peuvent être réunies par voie intra-opératoire.

3. Implant porteur selon l'une quelconque des revendications précédentes, **caractérisé en ce que** la première portion (46, 54, 74, 116) comprend une portion flexible continue qui peut être fixée à l'os et les segments (44, 51, 53, 72, 82, 84, 92, 102, 112) comprennent des segments de surface portante discrets qui peuvent être fixés sur la première portion (46, 54, 74, 116) et comprenant une surface portante (42, 52, 114) qui peut être engagée avec un composant de joint dans une relation de joint articulé.

4. Implant porteur selon l'une quelconque des revendications précédentes, **caractérisé en ce que** chacun de la pluralité de segments discrets (44, 51, 53, 72, 82, 84, 92, 102, 112) est capable de pivoter par rapport au premier matériau, chaque segment (44, 51, 53, 72, 82, 84, 92, 102, 112) étant orientable perpendiculairement à une surface du premier composant de joint en aboutement.

5. Implant porteur selon l'une quelconque des revendications précédentes, **caractérisé en ce que** la pluralité de segments discrets comprend des rubans allongés (92, 102).

6. Implant porteur selon l'une quelconque des revendications précédentes, **caractérisé en ce que** la pluralité de segments comprend un segment central (82) et une pluralité de segments radiaux (84) s'étendant vers l'extérieur depuis le segment central (82).

7. Implant porteur selon l'une quelconque des revendications précédentes, **caractérisé en ce que** la pluralité de segments discrets comprend une pluralité de segments allongés (92, 102) agencés parallèlement les uns aux autres avec des intervalles allongés (94) entre les segments.

8. Implant porteur selon l'une quelconque des revendications précédentes, **caractérisé en ce que** la pluralité de segments discrets comprend une pluralité de segments en serpentin (102).

9. Implant porteur selon l'une quelconque des revendications précédentes, **caractérisé en ce que** la pluralité de segments comprend une pluralité de segments semblables à des plaques (112) en chevauchement.
